# EUROPÄISCHE PATENTSCHRIFT

(11) **EP 0 328 860 B1**
(45) Veröffentlichungstag und Bekanntmachung des Hinweises auf die Patenterteilung: **24.03.1993**
(21) Anmeldenummer: 89100145.5
(22) Anmeldetag: 05.01.1989
(51) Int. Cl.: G01N 33/20, G01K 7/02

(54) **Metallisches Kontaktstück für eine Messlanze zur Durchführung von Messungen in Metallschmelzen**
Metallic contact piece for a measuring lance for measuring operations in molten metals
Pièce métallique de contact pour une lance de mesure pour faire des mesures dans les métaux en fusion

(30) Priorität: 17.02.1988 DE 3804880
(43) Veröffentlichungstag der Anmeldung: 23.08.1989
(73) Patentinhaber: Heraeus Electro-Nite International N.V., 2000 Antwerpen (BE)
(72) Erfinder: Knevels, Johan Maria Joseph, B-3690 Bree (BE)
(74) Vertreter: Heinen, Gerhard, Dr.

(56) Entgegenhaltungen:
- US-A- 4 582 951
- US-A- 4 645 243
- RESEARCH DISCLOSURE, Nr. 288, April 1988, Seite 243, New York, US; R.W. EDWARDS: "Low coolant sensing probe with connector corrosion prevention"

## Beschreibung

Die Erfindung betrifft ein metallisches Kontaktstück, das am unteren Ende einer Meßlanze angeordnet ist und auf das zur Herstellung einer elektrischen Verbindung eine verbrauchbare Sonde aufstekbar ist, die Meßeinrichtungen zur Durchführung von Messungen in Metallschmelzen enthält, wobei das Kontaktstück aus zwei Rohren besteht, die dadurch miteinander verbunden sind, daß ein verjüngter Abschnitt des einen Rohres in die erweiterte Öffnung des anderen Rohres unter Zwischenlegen eines hülsenförmigen Isolationskörpers eingeschoben wird, die äußeren Wandabschnitte der beiden Rohre gegen einen Flansch des Isolationskörpers anliegen, der den gleichen Außendurchmesser hat wie das Kontaktstück und wobei in den erweiterten Innenraum des Endes des Kontaktstückes, das elektrisch mit der Sonde verbunden wird ein becherförmiger Isolationskörper eingesetzt ist, dessen Innenraum voneinander isolierte Kontakte enthält, die durch den Boden des Isolationskörpers mit durch das Kontaktstück nach außen geführten elektrischen Leitungen verbunden sind.

Derartige Meßlanzen werden zur Bestimmung der Sauerstoffaktivität und/oder der Temperatur von Stahlschmelzen eingesetzt. Dabei wurden in der Praxis Fehlmessungen und voneinander abweichende Meßergebnisse festgestellt, für die es zunächst keine Erklärung gab. Im Rahmen einer langwierigen Versuchsreihe wurde herausgefunden, daß die Verfälschung der Meßergebnisse auf Wassereinschlüsse zurückzuführen ist, die in den aus Pappe bestehenden Schutzrohren vorhanden sind, die die Meßsonde und das Kontakstück umgeben. Beim Eintauchen der Sonde in die Schmelze wird das Wasser frei und gelangt durch kapillare Öffnungen zwischen den Isolationskörpern und den metallischen Wandungen der Rohre des Kontaktstückes in dessen mit einer Füllmasse ausgefüllten Innenraum. Da wegen der unterschiedlichen Wärmeausdehnungskoeffizienten der Füllmasse und der Metallrohre eine einwandfreie Abdichtung zwischen diesen Teilen nicht möglich ist, kann die eingedrungene Feuchtigkeit unter ungünstigen Bedingungen bis zu den elektrischen Kontakten vordringen und die Meßergebnisse verfälschen.

Die Erfindung basiert auf dieser überraschenden Erkenntnis, die durch die Versuchsergebnisse bestätigt wurde. Ihr liegt die Aufgabe zugrunde, das an der Meßlanze angebrachte Kontaktstück so auszubilden und abzudichten, das Verfälschungen der Meßergebnisse durch eindringende Feuchtigkeit vermieden werden.

Ausgehend von einem metallischen Kontaktstück der eingangs beschriebenen Art, besteht die Erfindung darin, daß der hülsenförmige Isolationskörper gegenüber der Wandung des erweiterten Abschnittes des einen Rohres und der Wandung des verjüngten Abschnittes des anderen Rohres des Kontaktstückes und der becherförmige Isolationskörper gegenüber der Wandung des erweiterten Innenraumes des Rohrendes, das mit der Sonde verbunden wird, durch Einlegen von ringförmigen Dichtungen abgedichtet sind und dass der obere Abschnitt des lanzenseitigen Rohres mit einer Silikonmasse ausgefüllt ist, die die in üblicher Weise benutzte Füllmasse an dieser Stelle ersetzt.

Eine bevorzugte Ausführungsform der Erfindung sieht vor, daß das Kontakstück wie folgt ausgebildet ist:
a) Der verjüngte Abschnitt des einen Rohres des Kontaktstückes und der erweiterte Abschnitt des anderen Rohres sind mit stufenförmigen Absätzen versehen, die umlaufende Anlageflächen bilden;
b) der hülsenförmige Isolationskörper enthält an entsprechenden Stellen angeordnete stufenförmige Aussparungen, durch die umlaufende radiale Anlageflächen gebildet werden, die denen der Rohre gegenüberliegen, wobei zwischen den sich gegenüberliegenden Anlageflächen Dichtungsringe aus elastischem Material angeordnet sind, die beim Ineinanderstecken der beiden Rohre des Kontaktstückes verspannt werden;
c) der Boden des becherförmigen Isolationskörpers enthält an seinem dem Innenraum des Rohres zugewandten Ende eine stufenförmige umlaufende Aussparung, der eine entsprechender stufenförmiger Absatz der Innenwandung des Rohres gegenüberliegt, wobei zwei sich gegenüberliegende radiale anlageflächen gebildet werden, zwischen denen beim Einsetzen des becherförmigen Isolationskörpers in das Rohrende ein Dichtungsring aus elastischem Material verspannt wird.

Zweckmäßigerweise weist das mit dem Lanzenhalter verbundene Rohr im Bereich seines unteren Abschnittes die Erweiterung und das Rohr, an das die Meßsonde anschließbar ist, an seinem oberen Ende den verjüngten Abschnitt auf.

Eine weitere zweckmäßige Ausführungsform der Erfindungen sieht vor, daß der Boden der becherförmigen Hülse mit zwei zylindrischen, sich in den Hohlraum des Rohres erstreckenden Ansätzen versehen ist, die sich bis in den Innenraum der Hülse erstreckende Längsbohrungen entalten, daß die elektrischen Leitungen, die mit der Kontaktbuchse und dem Kontaktstift verbunden werden, durch Bohrungen der Ansätze geführt sind und daß jede Eintrittsöffnung von einem Schrumpfrohr überdeckt ist, das dicht and der Leitung und dem zylindrischen Ansatz anliegt.

Die Erfindung wird anhand der Zeichnung, auf der ein Ausführungsbeispiel dargestellt ist im einzelnen erläutert. Es zeigen:
- Fig. 1: eine schematische Ansicht einer Tauchmeßlanze,
- Fig. 2: einen Längsschnitt durch das Kontaktstück und
- Fig. 3: einen Längsschnitt durch den unteren Teil des Kontaktstückes in vergrößerter Darstellung.

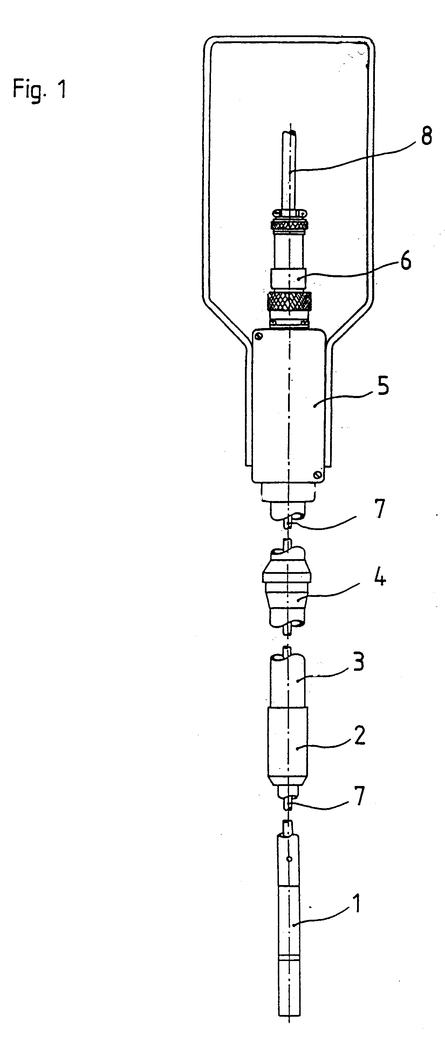

Die auf Fig. 1 dargestellte Tauchmeßlanze besteht aus dem Lanzenhalter (2), einem Stahlrohr (3), einem Anschlagstück (4), sowie dem Handgriff (5). Das in einer durchgehenden Innenbohrung isoliert geführte Kabel (7) ist am oberen Ende der Lanze über eine Schnellsteckverbindung (6) mit dem zu den Meßinstrumenten führenden Außenkabel (8) verbunden und am unteren Ende an ein an dem Lanzenhalter (2) befestigtes Kontakstück (1) angeschlossen, dessen besondere Ausbildung die Erfindung betrifft. Am Ende des Kontaktstückes (1) sitzt ein elektrischer Kontakt, mit dem eine austauschbare Meßsonde, z. B. zur Messung der Sauerstoffaktivität und/oder der Temperatur einer Metallschmelze abnehmbar elektrisch verbunden werden kann. Sowohl die Meßsonde als auch die Kontakthülse sind von einem oder mehreren meist aus Pappe bestehenden auf der Zeichnung nicht dargestellten Schutzrohren umgeben. Das äußere, das Kontaktstück (1) umgebende Papprohr kann über den Lanzenhalter (2) bis an das Anschlagstück (4) reichen.

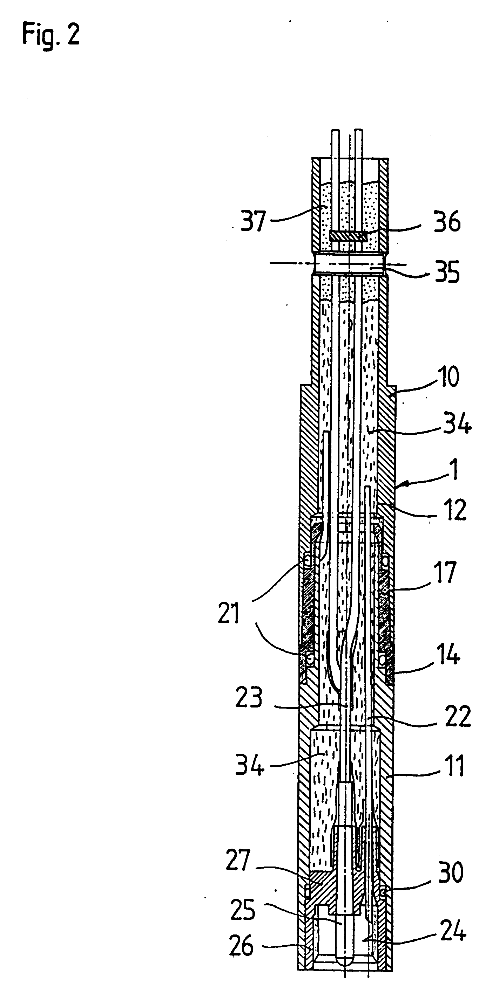

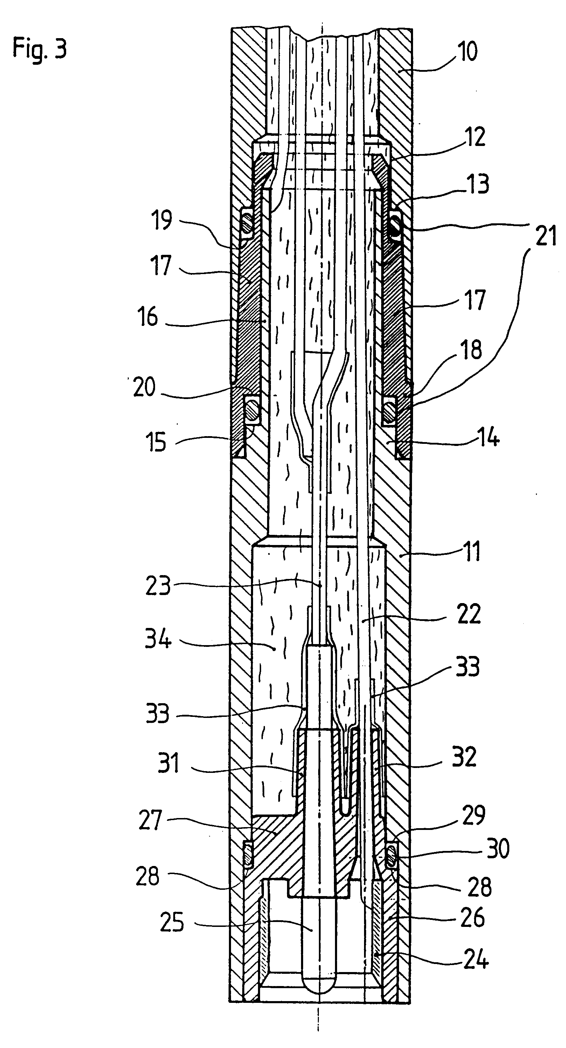

Wie sich aus den Fig. 2 und 3 ergibt, besteht das Kontakstück (1) aus zwei zylindrischen metallischen Rohren (10, 11), die hintereinander angeordnet und durch Ineinanderstecken miteinander verbunden sind.

Das obere lanzenseitige Rohr (10) ist mit der Meßlanze, das untere Rohr (11) elektrisch mit der Meßsonde verbunden. Der Hohlraum des Rohres (10) ist im Bereich seines unteren Endes erweitert und der erweiterte Abschnitt (12) mit einem sich in Richtung des Umfanges erstreckenden stufenförmigen Absatz (13) versehen, durch den eine umlaufende radiale Anlagefläche gebildet wird. Das obere Ende des Rohres (11), das den gleichen Außendurchmesser hat wie das Rohr (10) ist verjüngt; der verjüngte Abschnitt (14) ist mit einem Absatz (15) versehen, durch den dessen Durchmesser stufenförmig, unter Bildung einer umlaufenden radialen Anlagefläche verkleinert wird. Der oberhalb des Absatzes liegende Abschnitt (16) hat einen kleineren Außendurchmesser als der erweiterte Innenraum (12) des Rohres (10). Die Rohre (10, 11) werden durch Ineinanderstecken unter Zwischenlegen eines hülsenförmigen Isolationskörpers (17), der sich im Bereich der sich überlappenden Enden der Rohre (10, 11) erstreckt, miteinander verbunden. Der Isolationskörper ist mit einem unteren Flansch (18) versehen, der den gleichen Außendurchmesser wie die Rohre des Kontaktstückes (1) hat und gegen den die Enden der Rohre zur Anlage kommen. Erfindungsgemäß ist der Isolationskörper (17) mit stufenförmigen Aussparungen (19, 20) versehen, die den stufenförmigen Absätzen (13, 15) der Rohre (10, 11) entsprechen und so angeordnet sind, daß die durch sie gebildeten Anlageflächen denen der Rohre des Kontaktstückes gegenüberliegen. Zwischen die sich gegenüberliegenden Anlageflächen sind Dichtungsringe (21), z. B. O-Ringe aus elastischem Material eingelegt, die beim Ineinanderstecken der Rohre zusammengedrückt und gegen die sie umgebenden Wandungen verspannt werden. Auf diese Weise wird das Eindringen von Feuchtigkeit durch kapillare öffnungen zwischen den metallischen Teilen des Kontaktstückes und dem Isolationskörper mit einfachen Mitteln vermieden.

Wie sich aus Fig. 2 ergibt, sind zwei der vier Meßleitungen, die durch die Kontakthülse und die Meßlanze nach außen geführt und an die Meßinstrumente angeschlossen sind, nämlich die Leitungen (22, 23) elektrisch mit einem Kontakt verbunden, der aus einer Kontaktbuchse (24) und einem Kontaktstift (25) besteht. Der Kontakt ist im Innenraum einer becherförmigen Hülse (26) aus isolierendem Material angeordnet, die in das untere, der Meßsonde zugewandte Ende des Rohres (11) des Kontaktstückes eingeschoben und darin befestigt ist. Dabei ragt der Kontaktstift (25) mittig durch den Boden (27) der isolierenden Hüse (26), während die Kontaktbuchse (24) aus einer metallischen einlage besteht, die die seitliche Innenwandung der Hülse (26) bedeckt.

Um auch an dieser Stelle das Eindringen von Feuchtigkeit zu vermeiden, ist vorgesehen, daß der Boden (27) der becherförmigen Hülse (26) an seinem dem Hohlraum des Rohres (11) zugewandten Ende mit einer stufenförmigen nach innen verspringenden umlaufenden Aussparung (28) versehen ist, der eine stufenförmiger Absatz (29) der Innenwandung des Rohres (11) gegenüberliegt. Zwischen den so gebildeten radialen Anlageflächen ist ein Dichtungsring (30) aus elastischem Material eingelegt, der beim Einsetzen der becherförmigen Hülse verspannt wird (Fig. 3).

Eine bevorzugte Ausführungsform der Erfindung sieht vor, daß der Boden (27) der becherförmigen Hülse (26) mit zwei zylindrischen, sich in den Hohlraum des Rohres (11) erstreckenden Ansätzen (31, 32) versehen ist, die Längsbohrungen enthalten, die sich bis in den Innenraum der Hülse erstrecken. Der Ansatz (31) ist mittig angeordent und dient zur Aufnahme eines verlängerten Teils des Kontaktstiftes (25), während der zweite zylindrische Ansatz (32) exzentrisch angeordnet ist und zur Zuführung der elektrischen Leitung (22) zu der Kontaktbuchse (24) dient. Die Erfindung sieht vor, daß ein Abschnitt der elektrischen Leitungen (22, 23) vor deren Eintritt in die zylindrischen Ansätze (31, 32) von einem Schrumpfrohr (33) überdeckt ist, das dicht an den Leitungen anliegt und auch dicht über die zylindrischen Ansätze gezogen ist.

Der Hohlraum (34) der Rohre (10, 11) ist in üblicher Weise mit einer Füllmasse aus einem Zweikomponenten-Epoxyharz ausgefüllt. Mit (35) ist ein Lochdorn bezeichnet, mit (36) eine mit vier öffnungen versehene Distanzplatte, durch die die elektrischen Leitungen im Abstand voneinander gehalten werden. Es hat sich ferner als zweckmäßig erwiesen, einen mit (37) bezeichneten Abschnitt des Hohlraums des lanzenseitigen Endes des Rohres (10), der sich etwa bis in den Bereich des Lochdorns (35) erstreckt mit einer Siliconmasse auszufüllen, die das Eindringen von Feuchtigkeit in das obere Ende des Kontaktstückes vermeidet. Die Verwendung einer Siliconmasse anstelle einer üblichen Füllmasse ist in diesem Bereich möglich, weil sich das obere Ende des Kontaktstückes weniger erwärmt als dessen mittlerer und unterer Abschnitt.

## Patentansprüche

1. Metallisches Kontaktstück, das am unteren Ende einer Meßlanze angeordnet ist und auf das zur Herstellung einer elektrischen Verbindung eine verbrauchbare Sonde aufsteckbar ist, die Meßeinrichtungen zur Durchführung von Messungen in Metallschmelzen enthält, wobei das Kontaktstück aus zwei Rohren besteht, die dadurch miteinander verbunden sind, daß ein verjüngter Abschnitt des einen Rohres in die erweiterte Öffnung des anderen Rohres unter Zwischenlegen eines hülsenförmigen Isolationskörpers eingeschoben wird, die äußeren Wandabschnitte der beiden Rohre gegen einen Flansch des Isolationskörpers anliegen, der den gleichen Außendurchmesser hat wie das Kontaktstück und wobei in den erweiterten Innenraum des Endes des Kontaktstückes, das elektrisch mit der Sonde verbunden wird ein becherförmiger Isolationskörper eingesetzt ist, dessen Innenraum voneinander Isolierte Kontakte enthält, die durch den Boden des Isolationskörper mit durch das Kontaktstück nach außen geführten elektrischen Leitungen verbunden sind, dadurch gekennzeichnet, daß der hülsenförmige Isolationskörper (17) gegenüber der Wandung des erweiterten Abschnittes (12) des einen Rohres (10) und der Wandung des verjüngten Abschnittes (14) des anderen Rohres (11) des Kontaktstückes (1) und der becherförmige Isolationskörper (26) gegenüber der Wandung des erweiterten Innenraumes des Rohrendes, das mit der Sonde verbunden wird, durch Einlegen von ringförmigen Dichtungen (21, 30) abgedichtet sind und daß der obere Abschnitt (37) des lanzenseitigen Rohres (10) mit einer Silikonmasse ausgefüllt ist, die die in üblicher Weise benutzte Füllmasse an dieser Stelle ersetzt.

2. Metallisches Kontaktstück nach Anspruch 1, gekennzeichnet durch die folgenden Merkmale:
a) Der verjüngte Abschnitt (14) des einen Rohres (11) des Kontaktstückes (1) und der erweiterte Abschnitt (12) des anderen Rohres (10) sind mit stufenförmigen Absätzen (15, 13) versehen, die umlaufende Anlageflächen bilden;
b) der hülsenförmige Isolationskörper (17) enthält an entsprechenden Stellen angeordnete stufenförmige Aussparungen (19, 20), durch die umlaufende radiale Anlageflächen gebildet werden, die denen der Rohre gegenüberliegen, wobei zwischen den sich gegenüberliegenden Anlageflächen Dichtungsringe (21) aus elastischem Material angeordnet sind, die beim Ineinanderstecken der beiden Rohre des Kontaktstückes verspannt werden;
c) der Boden (27) des becherförmigen Isolationskörpers (26) enthält an seinem dem Innenraum des Rohres (11) zugewandten Ende eine stufenförmige umlaufende Aussparung (28), der ein entsprechender stufenförmiger Absatz (29) der Innenwandung des Rohres (11) gegenüberliegt, wobei zwei sich gegenüberliegende radiale Anlageflächen gebildet werden, zwischen denen beim Einsetzen des becherförmigen Isolationskörpers in das Rohrende ein Dichtungsring (30) aus elastischem Material verspannt wird.

3. Metallisches Kontaktstück nach den Ansprüchen 1 und 2, dadurch gekennzeichnet, daß das mit den Lanzenhalter (2) verbundene Rohr (10) im Bereich seines unteren Abschnittes (12) die Erweiterung und daß das Rohr (11), an das die Meßsonde anschließbar ist, an seinem oberen Ende den verjüngten Abschnitt (14) aufweist.

4. Metallisches Kontaktstück nach den Ansprüchen 1 bis 3, dadurch gekennzeichnet, daß der Boden (27) der becherförmigen Hülse (26) mit zwei zylindrischen, sich in den Hohlraum des Rohres (11) erstreckenden Ansätzen (31, 32) versehen ist, die sich bis in den Innenraum der Hülse erstreckende Längsbohrungen enthalten.

5. Metallisches Kontaktstück nach den Ansprüchen 1 bis 4, dadurch gekennzeichnet, daß die elektrischen Leitungen (22, 23), die mit der Kontaktbuchse (24) und dem Kontaktstift (25) verbunden werden, durch die Bohrungen der Ansätze (31, 32) geführt sind und daß jede Eintrittsöffnung von einem Schrumpfrohr (33) überdeckt ist, das dicht an der Leitung und dem zylindrischen Ansatz anliegt.

## Claims

1. Metal contact piece which is arranged at the lower end of a measuring lance and to which can be attached a disposable probe for producing an electrical connection, which contains measuring means for measuring operations in molten metals, wherein the contact piece consists of two tubes which are connected to each other by the fact that a tapering section of one tube is inserted into the expanded opening of the other tube by interposing a sleeve-shaped insulation body, the outer wall sections of the two tubes rest against a flange of the insulation body which has the same external diameter as the contact piece and in which into the expanded inner chamber at the end of the contact piece, which is electrically connected with the probe, is inserted a cup-shaped insulation body the inner chamber of which contains contacts, which are insulated from each other, which is connected through the base of the electrical insulation body with electrical conductors passing through the contact piece to the outside, **characterised** in that by inserting annular seals (21,30) the sleeve-shaped insulation body (17) is sealed against the wall of the expanded section (12) of one of the tubes (10) and the wall of the tapering Section (14) of the other tube (11) of the contact piece (1) and the cup-shaped insulation body (26) is sealed against the wall of the expanded inner chamber of the tube end which is in contact with the probe, and that the upper section (37) of the tube (10) on the side of the lance is filled with a silicone mass which replaces the filler mass normally used in this position.

2. Metal contact piece according to claim 1, **characterised** by the following features:
a) the tapered section (14) of a tube (11) of the contact piece (1) and the expanded section (12) of the other tube (10) are provided with step-like shoulders (15,13) which form circumferential contact surfaces;
b) the sleeve-shaped insulation body (17) at corresponding places contains step-like recesses (19,20) which are formed by circumferential, radial contact surfaces which are arranged opposite to those of the tubes, in which between the oppositely arranged contact surfaces there are arranged sealing rings (21) of elastic material which are stressed during the insertion of the two tubes of the contact piece;
c) the base (27) of the cup-shaped insulation body (26) at its end facing the inner chamber of the tube (11) contains a step-like circumferential recess (28) which is faced by a corresponding step-like shoulder (29) of the inner wall of the tube (11), in which there are formed two oppositely arranged radial contact surfaces between which a sealing ring (30) of elastic material is stressed during the insertion of the cup-shaped insulation body into the tube end.

3. Metal contact piece according to one of the claims 1 and 2, **characterised** in that the tube (10), connected to the lance holder (2), in the region of its lower section (12) has the expansion and that the tube (11), to which the measuring probe can be connected, in its upper end has the tapered section (14).

4. Metal contact piece according to claims 1 to 3, **characterised** in that the base (27) of the cup-shaped sleeve (26) is provided with two cylindrical projections (31,32), extending into the hollow chamber of the tube (11), which contain longitudinal drillings extending into the inner chamber of the sleeve.

5. Metal contact pieces according to the claims 1 to 4, **characterised** in that the electrical conductors (22,23), which are connected with the contact bush (24) and the contact pin (25), are passed through the drillings of the projections (31,32) and that each inlet opening is covered by a shrink fit tube (33) which rests tightly against the conductor and the cylindrical projection.

## Revendications

1. Pièce métallique de contact qui est disposée à l'extrémité inférieure d'une lance de mesure et sur laquelle, pour réaliser des liaisons électriques, on peut enficher une sonde consommable qui contient les dispositifs de mesure pour exécuter des mesures dans des métaux en fusion, étant précisé que la pièce de contact est constituée de deux tubes qui sont reliés l'un, à l'autre par le moyen qu'un tronçon rétréci du premier tube s'enfile dans l'ouverture élargie de l'autre tube, avec interposition d'un corps isolant en forme de douille, que les tronçons de paroi extérieure des deux tubes s'appuient contre un rebord du corps isolant qui a le même diamètre extérieur que la pièce de contact et étant précisé que dans l'espace intérieur, élargi, de l'extrémité de la pièce de contact, qui est électriquement reliée avec la sonde, est inséré un corps isolant en forme de boisseau dont l'espace intérieur contient des contacts qui sont isolés l'un de l'autre et qui, à travers le fond du corps isolant, sont reliés avec des conducteurs électriques qui sont guidés vers l'extérieur à travers la pièce de contact, pièce métallique de contact caractérisée en ce que sont rendus étanches, par insertion de garnitures d'étanchéité de forme annulaire (21, 30), le corps isolant en forme de douille (17) à l'égard de la paroi du tronçon élargi (12) du premier tube (10) et à l'égard de la paroi du tronçon rétréci (14) de l'autre tube (11) de la pièce de contact (1), ainsi que le corps isolant en forme de boisseau (26) à l'égard de la paroi de l'espace intérieur élargi de l'extrémité du tube, qui est reliée avec la sonde, et en ce que le tronçon supérieur (37) du tube (10), du côté de la lance, est rempli d'une masse de silicone qui remplace à cet endroit la masse de remplissage utilisée de façon habituelle.

2. Pièce métallique de contact selon la revendication 1, caractérisée par les caractéristiques suivantes:
a) le tronçon rétréci (14) du premier tube (11) de la pièce de contact (1) et le tronçon élargi (12) de l'autre tube (10) présentent des décrochements en forme d'échelon (15, 13) qui forment des surfaces d'appui périphériques;
b) le corps isolant en forme de douille (17) contient des évidements en forme d'échelon (19, 20) qui sont disposés en des endroits appropriés et par lesquels sont formées des surfaces radiales périphériques d'appui qui sont situées en face de celles du tube, étant précisé qu'entre les surfaces d'appui, situées en face l'une de l'autre, sont disposées des bagues d'étanchéité (21) en matériau élastique qui, lors de l'enfichagé des deux tubes de la pièce de contact l'un dans l'autre, sont mises sous contrainte.
c) le fond (27) du corps isolant en forme de boisseau (26) contient, à son extrémité orientée vers l'espace intérieur (11), un évidement périphérique en forme d'échelon (28) qui est situé en face d'un décrochement correspondant, en forme d'échelon, (29), de la paroi intérieure du tube (11), ce qui forme deux surfaces d'appui radiales, situées en face l'une de l'autre, entre lesquelles, lors de l'insertion du corps isolant en forme de boisseau dans l'extrémité du tube, une bague d'étanchéité (30) en matériau élastique est mise sous contrainte.

3. Pièce métallique de contact selon les revendications 1 et 2, caractérisée par le fait que le tube (10) relié au porte-lance (2) présente l'élargissement dans la zone de son tronçon inférieur (12) et que le tube (11) auquel peut se relier la sonde de mesure présente le tronçon rétréci (14) à son extrémité supérieure.

4. Pièce métallique de contact selon les revendications 1 à 3, caractérisée par le fait que le fond (27) de la douille en forme de boisseau (26) présente deux appendices cylindriques (31, 32) qui s'étendent dans l'espace creux du tube (11) et contiennent des perçages longitudinaux s'étendant jusque dans l'espace intérieur de la douille.

5. Pièce métallique de contact selon les revendications 1 à 4, caractérisée par le fait que les conducteurs électriques (22, 23) qui sont reliés avec la fiche femelle de contact (24) et avec la broche de contact (25) sont guidés à travers les perçages des appendices (31, 32) et que chaque ouverture d'entrée est recouverte d'un tube rétractable (33) qui s'appuie, avec étanchéité, contre le conducteur et contre l'appendice cylindrique.
